# EUROPEAN PATENT APPLICATION

(11) **EP 4 559 444 A1**
(43) Date of publication of application: **28.05.2025**
(21) Application number: 23211997.4
(22) Date of filing: 24.11.2023
(51) Int. Cl.: A61F 7/02, A42B 1/008, A61F 7/00

(54) **PAIN-RELIEVING HEADWEAR**

(71) Applicant: Relevo Technology ApS, 3460 Birkerød (DK)
(72) Inventor: QUISTGAARD KØLBÆK, Rebekka, 3460 Birkerød (DK)
(74) Representative: COPA Copenhagen Patents

(57) **Abstract**

Disclosed is an assembly of gel packs comprising a plurality of gel packs and a headwear comprising the assembly of gel packs. Each of the plurality of gel packs comprises a liquid or a gel having a specific heat capacity sufficient to provide cooling or heating after being cooled or heated, and wherein each of the plurality of gel packs have a first side and an opposite second side, the first side joining the opposite second side along a rim defining a substantially planar shape of the respective gel pack. The plurality of gel packs comprising a first primary gel pack and a first secondary gel pack, wherein the substantially planar shape of the first primary gel pack is a first primary shape and the substantially planar shape of the first secondary gel pack is a first secondary shape different than the first primary shape.

## Description

The present disclosure relates to a pain-relieving object, such as a pain-relieving headwear. More specifically, the present disclosure relates to a head worn object for relieving pain, e.g. caused by migraine, by cooling and/or heating.

### BACKGROUND

Most people have tried the experience of having headache and knows that it can be both painful and disabling. However, it should be clear that some, e.g. people suffering from migraine, experience headaches more often, more painful and more disabling than others.

Sometimes headaches can be attributed certain reasons or occurs under certain conditions, for example, many experience headaches in connection with menopause, concussions, hormonal disturbances, etc. However, in other cases, headaches may occur without any clear reason. In addition, there are several different headache types such as migraine, tension headache, post-traumatic headache, medication overuse headache, cluster headache (also known as Horton's headache), trigeminal neuralgia or idiopathic intracranial hypertension.

Whatever the reason, people suffering from headache is logically seeking relief for their pain. However, providing pain relief for headaches not only has a personal benefit for the one suffering from the headache, it also helps the person to attend to their daily tasks, in effect contributing to their own wellbeing as well as to that of their family and the society. For example, according to a report from October 2015, the Danish Health Authority estimates that migraine costs DKK 420 million each year in treatments and DKK 1.2 billion in lost production.

Studies have shown that cooling can efficiently relieve the pain of migraines and headaches, and cooling has been a common non-invasive form of headache relief. For some, heat has also been found to have a soothing effect. Thus, having an object, such as a headwear, which allows cooling or heating of the head is appreciated by the person suffering from headaches. However, providing such object, which both provides the desired temperature altering effect, while also being comfortable and easy to store and use is not completely straightforward.

### SUMMARY

It is an object of the present disclosure to provide an easier, more convenient, and possibly more efficient pain-relieving headwear, allowing cooling and/or heating of the head of the person suffering from a headache.

Thus, the present disclosure relates to an assembly of gel packs comprising a plurality of gel packs for cooling or heating of selective regions of a user's head and a headwear comprising the assembly of gel packs.

It is an advantage of the present disclosure, that it provides an assembly of gel packs and a headwear, where the user may select which regions of the head the user wants to cool or heat. More specifically, with the present disclosure, the user is given the possibility to select cooling or heating of each of a number of different anatomical regions of the brain and skull. It is a further advantage of the present disclosure that a headwear and an assembly of gel packs are provided, where cooling and/or heating may be provided throughout the head of the user. Thus, the present disclosure balances the need of providing the ability to cool and/or heat all or selected regions of the user's head while keeping the number of different parts low.

### GENERAL DESCRIPTION

The disclosed assembly of gel packs comprises a plurality of gel packs, wherein each of the plurality of gel packs comprises a liquid or a gel having a specific heat capacity sufficient to provide cooling or heating after being cooled or heated (e.g. in the refrigerator or microwave, respectively). The plurality of gel packs may be at least 6 gel packs, such as at least 7 gel packs. In some examples, the plurality of gel packs may be between 6-15 gel packs, such as between 7-10 gel packs, such as 7-8 gel packs.

Each of the plurality of gel packs further has a first side and an opposite second side. The first side joins the opposite second side along a rim defining a substantially planar shape of the respective gel pack. The rim may be formed by welding of the first side and the second side. The first side and/or the second side may be made from polyethylene terephthalate (PET) (e.g. Dacron) and/or optionally one or more other materials, such as polyvinyl chloride (PVC).

The disclosed headwear comprises the assembly and/or the plurality of gel packs of the assembly, for cooling or heating of selective regions of a user's head.

The headwear has an inside fabric layer and an opposite outside fabric layer. The inside fabric layer is adapted to be arranged towards the head of the user when wearing the headwear. The headwear further comprises a plurality of pockets formed between the inside fabric layer and the outside fabric layer. Each of the plurality of pockets is adapted to contain a corresponding gel pack of the plurality of gel packs.

The plurality of gel packs comprises a first primary gel pack and a first secondary gel pack. The plurality of gel packs may comprise a second primary gel pack and/or a second secondary gel pack.

The plurality of gel packs may comprise a third primary gel pack, a third secondary gel pack, and/or a third tertiary gel pack. The plurality of gel packs may comprise a fourth gel pack.

The plurality of pockets of the headwear may comprise a first primary pocket adapted to contain the first primary gel pack. The plurality of pockets of the headwear may comprise a first secondary pocket adapted to contain the first secondary gel pack. The plurality of pockets of the headwear may comprise a second primary pocket adapted to contain the second primary gel pack. The plurality of pockets of the headwear may comprise a second secondary pocket adapted to contain the second secondary gel pack. The plurality of pockets of the headwear may comprise a third primary pocket adapted to contain the third primary gel pack. The plurality of pockets of the headwear may comprise a third secondary pocket adapted to contain the third secondary gel pack. The plurality of pockets of the headwear may comprise a third tertiary pocket adapted to contain the third tertiary gel pack. The plurality of pockets of the headwear may comprise a fourth pocket adapted to contain the fourth gel pack.

The substantially planar shape of the first primary gel pack is a first primary shape. The substantially planar shape of the first secondary gel pack is a first secondary shape. The substantially planar shape of the second primary gel pack is a second primary shape. The substantially planar shape of the second secondary gel pack is a second secondary shape. The substantially planar shape of the third primary gel pack is a third primary shape. The substantially planar shape of the third secondary gel pack is a third secondary shape. The substantially planar shape of the third tertiary gel pack is a third tertiary shape. The substantially planar shape of the fourth gel pack is a fourth shape.

The first primary shape may be not rectangular. For example, the first primary shape may be arch shaped. The second primary shape may be the same as the first primary shape, i.e. the substantially planar shape of the second primary gel pack may be the first primary shape. The first secondary shape is different than the first primary shape. The first secondary shape may be not rectangular. For example, the first secondary shape may be a pentagonal shape, e.g. a trapezoid with a cut corner. The second secondary shape may be the same as the first secondary shape, i.e. the substantially planar shape of the second secondary gel pack may be the first secondary shape. The third primary shape may be substantially rectangular. The third secondary shape may be substantially rectangular. The third tertiary shape may be substantially rectangular. The first primary shape, the first secondary shape, the third primary shape, the third secondary shape and the third tertiary shape may all be different. The fourth shape may be kidney shaped or bean shaped.

The first primary shape may have a first edge side with a convex curvature and an opposite second edge side with a concave curvature. The radius of curvature of the first edge side may be larger than the radius of curvature of the second edge side. The radius of curvature of the first edge side may be between 75-150 mm, such as between 90-145 mm, such as between 100-125 mm. The radius of curvature of the second edge side may be between 30-50 mm, such as between 35-45 mm, such as about 40 mm. The first primary shape may have a width between 150-230 mm, such as between 170-210 mm, such as between 180-200 mm and/or a height (perpendicular to the width) between 100-160 mm, such as between 120-140 mm. The first primary shape as disclosed has the advantage that it may provide for cooling or heating of the temporal lobe of the user's head.

The first secondary shape may have a first edge side and an opposite second edge side. The first edge side may be substantially straight. The first edge side may have a length between 100-130 mm, such as between 110-120 mm. The second edge side may be non-parallel with the first edge side. The second edge side may be substantially straight and/or may have a minor concave curvature, e.g. the second edge side may have a radius of curvature of more than 200 mm, such as between 200-400 mm.

The first secondary shape may have a third edge side between the first edge side and the second edge side of the first secondary shape. The third edge side may be substantially straight. The third edge side may be substantially perpendicular to the first edge side of the first secondary shape. The third edge side may be substantially perpendicular to the first edge side. The third edge side may have a length between 70-110 mm, such as between 85-95 mm.

The first secondary shape may have a fourth edge side between the first edge side and the second edge side of the first secondary shape. The fourth edge side may be substantially straight. The fourth edge side may be opposite the third edge side of the first secondary shape. The fourth edge side may be non-parallel with the third edge side of the first secondary shape. The fourth edge side may have a length between 60-90 mm, such as between 70-80 mm.

The first secondary shape may have a fifth edge side between the first edge side and the fourth edge side of the first secondary shape. The fifth edge side may be opposite the third edge side of the first secondary shape. The fifth edge side may be concave. The fifth edge side may have a radius of curvature between 50-70 mm, such as between 55-65 mm.

The first secondary shape as disclosed has the advantage that it may provide for cooling or heating of the neck muscles, which may be a frequent origin for headaches.

The third primary gel pack, the third secondary gel pack and/or the third tertiary gel pack may be adapted to cool or heat along a centre line of the user's head. For example, the third primary gel pack may be adapted to cool or heat the occipital lobe of the user's head, the third secondary gel pack may be adapted to cool or heat the parietal lobe of the user's head, and/or the third tertiary gel pack may be adapted to cool or heat the frontal lobe of the user's head.

The third primary gel pack may have a third primary shape. The third primary shape may be a substantially rectangular shape. The third primary shape may have a first edge side and an opposite second edge side. The third primary shape may have a third edge side between the first edge side and the second edge side. The third primary shape may have a fourth edge side between the first edge side and the second edge side. The fourth edge side may be opposite the third edge side. The first edge side and the second edge side may be parallel. The third edge side and the fourth edge side may be parallel. The first edge side and the second edge side may be perpendicular to the third edge side and the fourth edge side.

The first edge side and the second edge side of the third primary shape may have a length between 90-120 mm, such as between 100-110 mm. The third edge side and the fourth edge side of the third primary shape may have a length between 70-110 mm, such as between 85-95 mm.

The third secondary gel pack may have a third secondary shape. The third secondary shape may be a substantially rectangular shape. The third secondary shape may have a first edge side and an opposite second edge side. The third secondary shape may have a third edge side between the first edge side and the second edge side. The third secondary shape may have a fourth edge side between the first edge side and the second edge side. The fourth edge side may be opposite the third edge side. The first edge side and the second edge side may be parallel. The third edge side and the fourth edge side may be parallel. The first edge side and the second edge side may be perpendicular to the third edge side and the fourth edge side.

The first edge side and the second edge side of the third secondary shape may have a length between 140-170 mm, such as between 150-160 mm. The third edge side and the fourth edge side of the third secondary shape may have a length between 70-110 mm, such as between 85-95 mm.

The third tertiary gel pack may have a third tertiary shape. The third tertiary shape may be a substantially rectangular shape. The third tertiary shape may have a first edge side and an opposite second edge side. The third tertiary shape may have a third edge side between the first edge side and the second edge side. The third tertiary shape may have a fourth edge side between the first edge side and the second edge side. The fourth edge side may be opposite the third edge side. The first edge side and the second edge side may be parallel. The third edge side and the fourth edge side may be parallel. The first edge side and the second edge side may be perpendicular to the third edge side and the fourth edge side.

The first edge side and the second edge side of the third tertiary shape may have a length between 190-235 mm, such as between 205-220 mm. The third edge side and the fourth edge side of the third tertiary shape may have a length between 70-110 mm, such as between 85-95 mm.

The fourth gel pack may be adapted to cool or heat around the user's eyes. The fourth gel pack may have a fourth shape. The fourth shape may be kidney shaped or bean shaped. The fourth shape may have a first edge side and an opposite second edge side. The first edge side may have a convex curvature. The second edge side may have a concave curvature. The second edge side may be substantially straight. The radius of curvature of the first edge side may be smaller than the radius of curvature of the second edge side. The radius of curvature of the first edge side may be between 70-100 mm, such as between 80-90 mm. The radius of curvature of the second edge side may be more than 500 mm, such as more than 800 mm.

The fourth shape may have a height being the maximum distance measured from and perpendicular to the second edge side. The height of the fourth shape may be between 50-95 mm, such as between 65-80 mm. The fourth shape may have a width being the maximum distance of the fourth shape measured perpendicular to the height, e.g. substantially parallel to the second edge side. The width of the fourth shape may be between 150-210 mm, such as between 170-190 mm.

One or more of the plurality of gel packs may comprise one or more dividing paths subdividing the respective gel pack into two or more sub-compartments, such as two sub-compartments and/or three sub-compartments. The one or more dividing paths may restrict (in some examples without completely preventing) flow of the liquid or gel between two adjacent sub-compartments. The one or more dividing paths may be formed by welding together the first side and the second side of the one or more gel packs along respective paths to form the one or more dividing paths.

For example, the first primary gel pack may comprise one or two dividing paths subdividing the first primary gel pack into two or three sub-compartments. The first secondary gel pack may comprise one dividing path subdividing the first secondary gel pack into two sub-compartments. The second primary gel pack may comprise one or two dividing paths subdividing the second primary gel pack into two or three sub-compartments. The second secondary gel pack may comprise one dividing path subdividing the second secondary gel pack into two sub-compartments.

The third primary gel pack may comprise one dividing path subdividing the third primary gel pack into two sub-compartments. The third secondary gel pack may comprise one dividing path subdividing the third secondary gel pack into two sub-compartments. The third tertiary gel pack may comprise one or two dividing paths subdividing the third tertiary gel pack into two or three sub-compartments.

The fourth gel pack may be devoid of any dividing paths.

The liquid or gel of the plurality of gel packs may have a specific heat capacity of at least 2500 J/(kg-K), such as at least 3000 J/(kg-K), such as at least 3500 J/(kg-K). The liquid or gel of the plurality of gel packs may have a freezing point below 0 degrees Celsius. For example, the liquid or gel of the plurality of gel packs may have a freezing point below -10 degrees Celsius.

The liquid or gel of the plurality of gel packs may be a hydrogel. Alternatively, or additionally, the liquid or gel of the plurality of gel packs may comprise more than 70% water and/or more than 15% propylene glycol.

Each of the plurality of pockets may have a pocket opening to allow insertion and removal of the gel packs from the pocket. The pocket opening may be through the inside fabric layer. Thus, insertion and removal of the gel packs are performed via the inside of the headwear. This may reduce the risk of the gel packs falling out of their pockets when the headwear is being worn. Each of the plurality of pockets may have an inside overlap, e.g. at the pocket opening, adapted to restrict removal of the gel pack from the pocket. Thereby, the risk of unintentional removal of a gel pack from its pocket may be even more reduced.

The outside fabric layer may be made of neoprene. Thereby, an insulating layer is provided on the outside of the gel packs, whereby the cooling and/or heating effect on the user's head is improved. The inside fabric layer may comprise graphene. Graphene in the inside fabric layer improves heat conductivity improving the cooling and/or heating effect of the gel packs on the user's head.

### BRIEF DESCRIPTION OF THE FIGURES

Embodiments of the disclosure will be described in more detail in the following with regard to the accompanying figures. The figures show one way of implementing the present disclosure and are not to be construed as being limiting to other possible embodiments falling within the scope of the attached claim set.
Fig. 1 schematically illustrates a user wearing an exemplary headwear,
Fig. 2 schematically illustrates an exemplary headwear,
Fig. 3 schematically illustrates a cross sectional view of a pocket and an exemplary gel pack,
Fig. 4 schematically illustrates an exemplary assembly of gel packs, and
Fig. 5 schematically illustrates individual gel packs.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

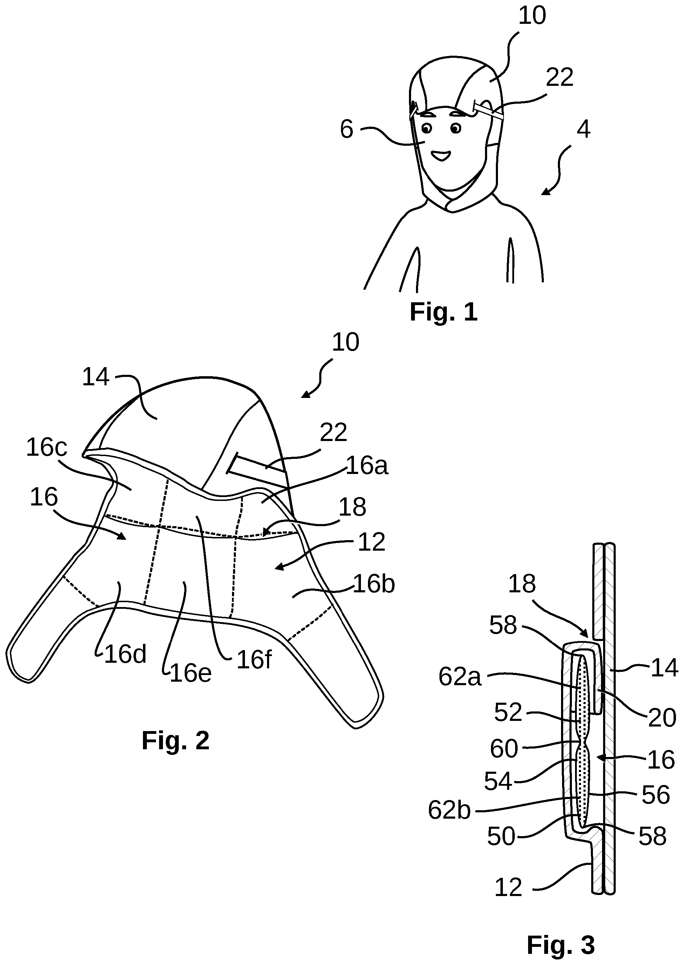

Fig. 1 schematically illustrates a user 4 wearing an exemplary headwear 10 on the head 6. The headwear 10 is further illustrated in Fig. 2. The headwear 10 is adapted to provide cooling or heating of the user's head 6, such as of selective regions of the user's head 6.

The headwear 10 has an inside fabric layer 12 and an opposite outside fabric layer 14. The inside fabric layer 12 is adapted to be arranged towards the head 6 of the user 4 when wearing the headwear 10, e.g. as illustrated in Fig. 1. The headwear 10 comprises a plurality of pockets 16 formed between the inside fabric layer 12 and the outside fabric layer 14. Each of the plurality of pockets 16 is adapted to contain a corresponding gel pack of a plurality of gel packs, which provides the cooling or heating effect of the headwear. The plurality of pockets 16 includes, in the illustrated example, a first primary pocket 16a, a first secondary pocket 16b, a second primary pocket 16c, a second secondary pocket 16d, a third primary pocket 16e, and a third secondary pocket 16f. As will be apparent from the further description, the plurality of pockets 16 may include further pockets, which is however not visible in the present illustration.

The headwear 10 further comprises one or more straps 22, e.g. Velcro straps, which may be used to tighten the headwear 10 to the head 6 of the user. For example, such that the front part of the headwear is brought into contact with the forehead of the user's head 6, and similarly the back of the headwear 10 may be brought into contact with the back of the user's head 6.

Although not specifically illustrated in Fig. 1, the headwear 10 may comprise a part for covering the eyes of the user 4, and the headwear 10 may comprise a corresponding pocket for receiving a gel pack for cooling or heating around the eyes of the user 4.

Fig. 3 schematically illustrates a cross sectional view of a pocket 16 and an exemplary contained gel pack 50. As illustrated, the pocket 16 is formed between the inside fabric layer 12 and the outside fabric layer 14. The pocket 16 has a pocket opening 18. The pocket opening 18 allows insertion and removal of the gel pack 50 from the pocket 16. In the illustrated example, the pocket opening 18 is provided through the inside fabric layer 12. When being provided through the inside fabric layer 12, as illustrated, the gel pack can be removed from and inserted into the pocket 16 from the inside of the headwear 10.

As also illustrated, the pocket 16 may have an inside overlap 20 at the pocket opening 18. The inside overlap 20 is adapted to restrict removal of the gel pack 50 from the pocket 16. However, since the gel pack 50 as well as the headwear 10 is relatively soft, the gel pack 50 may still be removed by the user, but the inside overlap 20 reduces the risk of the gel pack 50 inadvertently falling out of the pocket 16.

The gel pack 50 comprises a liquid or gel 52, which has a specific heat capacity sufficient to provide a cooling or heating effect after being respectively cooled (e.g. in the refrigerator) or heated (e.g. in the microwave oven). For example, the liquid or gel 52 may have a specific heat capacity of more than 2500 J/(kg·K). An exemplary liquid or gel 52 may be water mixed with propylene glycol. The liquid or gel 52 may be a hydrogel.

The gel pack 50 has a first side 54 and an opposite second side 56. The first side 54 joins the opposite second side 56 along a rim 58 defining a substantially planar shape of the gel pack 50.

The gel pack 50 comprises a dividing path 60, which subdivides the gel pack 50 into two sub-compartments 62a, 62b. The dividing path 60 restricts flow of the liquid or gel 52 between the two adjacent sub-compartments 62a, 62b. However, the dividing path 60 may not necessarily completely prevent the flow of the liquid or gel 52 between the sub-compartments 62a, 62b.

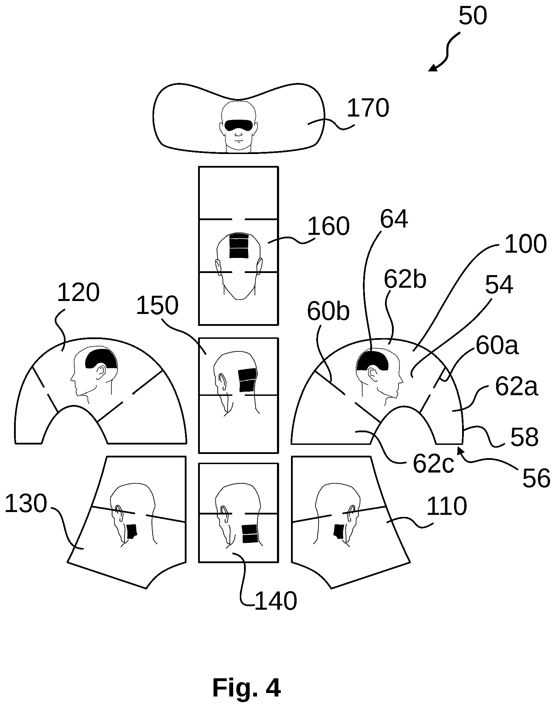

Fig. 4 schematically illustrates an exemplary assembly of gel packs 50 for cooling or heating of selective regions of a user's head. As described previously, each of the plurality of gel packs 50 comprises a liquid or a gel having a specific heat capacity sufficient to provide cooling or heating after being cooled or heated.

The plurality of gel packs 50, as illustrated, includes a first primary gel pack 100, a first secondary gel pack 110, a second primary gel pack 120, a second secondary gel pack 130, a third primary gel pack 140, a third secondary gel pack 150, a third tertiary gel pack 160, and a fourth gel pack 170. The gel packs 50 are adapted to be contained by the plurality of pockets 16 of the headwear 10 as illustrated in Fig. 2. Thus, each of the plurality of pockets 16 is adapted to contain a corresponding gel pack of the plurality of gel packs 50. For example, the headwear 10 may comprise a first primary pocket 16a shaped to contain the first primary gel pack 100. The headwear 10 may comprise a first secondary pocket 16b shaped to contain the first secondary gel pack 110. The headwear 10 may comprise a second primary pocket 16c shaped to contain the second primary gel pack 120. The headwear 10 may comprise a second secondary pocket 16d shaped to contain the second secondary gel pack 130. The headwear 10 may comprise a third primary pocket 16e shaped to contain the third primary gel pack 140. The headwear 10 may comprise a third secondary pocket 16f shaped to contain the third secondary gel pack 150. The headwear 10 may comprise a third tertiary pocket shaped to contain the third tertiary gel pack 160. The headwear 10 may comprise a fourth pocket shaped to contain the fourth gel pack 170.

The gel packs 50 may be shaped and divided to cover the entirety of the user's head, and in such a way that various regions may be selected for cooling or heating. For example, the user may wish to only cool the right temporal lobe, and in such case the user may use the headwear with only the first primary gel pack 100 in a cooled state. In the example illustrated, the assembly of gel packs 50 provides the user with the ability to selectively cool or heat one or more of the temporal lobes (with the first primary gel pack 100 and the second primary gel pack 120), the frontal lobe (with the third tertiary gel pack 160), the parietal lobe (with the third secondary gel pack 150), the occipital lobe (with the third primary gel pack 140), the neck muscles (with the first secondary gel pack 110 and the second secondary gel pack 130), and the region of the eyes (with the third gel pack 170).

As mentioned also in relation to Fig. 3, each of the plurality of gel packs 50 has a first side 54 and an opposite second side 58 (here illustrated for the first primary gel pack 100, but it should be understood that this similarly may apply for the other gel packs 110, 120, 130, 140, 150, 160, 170). The first side 54 joins the opposite second side 56 along a rim 58 defining a substantially planar shape of the gel pack 50. Fig. 4 schematically illustrates the planar shapes of the plurality of gel packs 50 as defined by their rims 58.

The substantially planar shape of the first primary gel pack 100 is a first primary shape, which, in the illustrated example, resembles an arch. The substantially planar shape of the first secondary gel pack 110 is a first secondary shape, which, in the illustrated example, resembles a trapezoid, with a cut corner.

The substantially planar shape of the second primary gel pack 120 is a second primary shape, which, in the illustrated example, resembles an arch. The first primary gel pack 100 and the second primary gel pack 120 may have the same shape, i.e. the second primary shape may be the same as the first primary shape. The substantially planar shape of the second secondary gel pack 130 is a second secondary shape, which, in the illustrated example, resembles a trapezoid, with a cut corner. The first secondary gel pack 110 and the second secondary gel pack 130 may have the same shape, i.e. the second secondary shape may be the same as the first secondary shape.

The substantially planar shape of the third primary gel pack 140 is a third primary shape, which, in the illustrated example, resembles a rectangle. The substantially planar shape of the third secondary gel pack 150 is a third secondary shape, which, in the illustrated example, resembles a rectangle. The substantially planar shape of the third tertiary gel pack 160 is a third tertiary shape, which, in the illustrated example, resembles a rectangle. The substantially planar shape of the fourth gel pack 170 is a fourth shape, which, in the illustrated example, resembles a kidney shape or bean shape.

As mentioned also in relation to Fig. 3, one or more of the plurality of gel packs 50 may comprise one or more dividing paths 60a, 60b (here illustrated for the first primary gel pack 100, but it should be understood that this similarly may apply for the other gel packs 110, 120, 130, 140, 150, 160, 170). The dividing paths 60a, 60b subdivides the gel pack 100 into two or more sub-compartments 62a, 62b, 62c. The dividing paths 60a, 60b restrict flow of the liquid or gel between adjacent sub-compartments 62a, 62b, 62c. As illustrated the dividing paths 60a, 60b may be only partly dividing the sub-compartments, such that the liquid or gel may still move between the sub-compartments, but the flow is restricted to avoid all of the liquid or gel to end up in one end of the gel pack 100. As seen, some of the gel packs 50 may comprise one dividing path and consequently two sub-compartments, while others may comprise two dividing paths and consequently three sub-compartments. It is also possible to omit the dividing path for some of the gel packs 50 resulting in only one compartment.

The gel packs 50 may comprise labels or icons 64 for recognising where the respective gel pack 50 is intended to be inserted in the headwear 10.

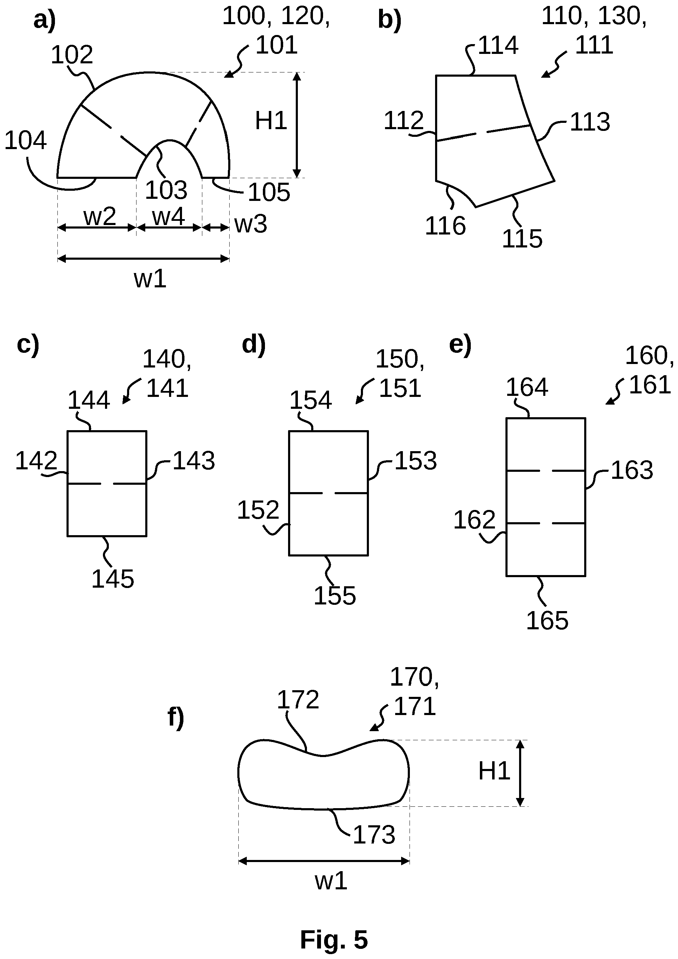

Fig. 5 schematically illustrates the individual gel packs 50 of the assembly of gel packs 50 as illustrated in Fig. 4.

Fig. 5a illustrates the first primary gel pack 100 and/or the second primary gel pack 120, because as mentioned previously, the first primary gel pack 100 and the second primary gel pack 120 may be the same shape, i.e. the first primary shape 101 (although it may be turned upside down when being used for the opposite side of the head).

The first primary shape 101 has a first edge side 102 with a convex curvature and an opposite second edge side 103 with a concave curvature. The concave curvature of the second edge side 103 makes room for the ear of the user and provides for the first primary gel pack 100 and the second primary gel pack 120 to provide cooling or heating to the temporal lobe of the user. The first edge side 102 may have a radius of curvature between 75-150 mm, such as between 90-145 mm, such as between 100-125 mm. The second edge side 103 may have a radius of curvature between 30-50 mm, such as between 35-45 mm, such as about 40 mm.

The first primary shape 101 further has a third edge side 104 between the first edge side 102 and the second edge side 103. The first primary shape 101 further has a fourth edge side 105 between the first edge side 102 and the second edge side 103. The third edge side 104 and the fourth edge side of the first primary shape 101 may be substantially parallel and/or coaxial, as illustrated.

A height H1 of the first primary shape 101 may be measured from the third edge side 104 and/or the fourth edge side 105 and denoting the maximum distance of the first primary shape 101 perpendicular to the third edge side 104 and/or the fourth edge side 105. The height H1 of the first primary shape 101 may be between 100-160 mm, such as between 120-140 mm.

A width W1 of the first primary shape 101 may be measured from the intersection between the third edge side 104 and the first edge side 102 to the intersection between the fourth edge side 105 and the first edge side 102. The width W1 may be measured perpendicular to the height H1. The width W1 may be between 150-230 mm, such as between 170-210 mm, such as between 180-200 mm. The width W2 of the third edge side 104 may be between 70-110 mm, such as between 80-95 mm. The width W3 of the fourth edge side 105 may be between 15-40 mm, such as between 25-30 mm. The width W4 between the third edge side 104 and the fourth edge side 105, such as the width of the second edge side 103 parallel to the third edge side 104 and the fourth edge side 105, may be between 40-100 mm, such as between 60-80 mm.

Fig. 5b illustrates the first secondary gel pack 110 and/or the second secondary gel pack 130, because as mentioned previously, the first secondary gel pack 110 and the second secondary gel pack 130 may be the same shape, i.e. the first secondary shape 111 (although it may be turned upside down when being used for the opposite side of the head).

The first secondary shape 111 has a first edge side 112 being substantially straight and an opposite second edge side 113 being non-parallel with the first edge side 112. The second edge side 113 may be substantially straight and/or have a minor concave curvature, e.g. having a radius of curvature of more than 200 mm, such as between 200-400 mm. The first edge side 112 may have a length between 100-130 mm, such as between 110-120 mm.

The first secondary shape 111 has a third edge side 114 between the first edge side 112 and the second edge side 113. The third edge side 114 is substantially straight. The third edge side 114 is substantially perpendicular to the first edge side 112. The third edge side 114 may have a length between 70-110 mm, such as between 85-95 mm.

The first secondary shape 111 has a fourth edge side 115 between the first edge side 112 and the second edge side 113. The fourth edge side 115 may be substantially straight. The fourth edge side 115 is opposite the third edge side 114. The fourth edge side 115 is non-parallel with the third edge side 114. The fourth edge side 115 may have a length between 60-90 mm, such as between 70-80 mm.

The first secondary shape 111 has a fifth edge side 116 between the first edge 112 side and the fourth edge side 115. The fifth edge side 116 is opposite the third edge side 114. The fifth edge side 116 is concave. The fifth edge side may have a radius of curvature between 50-70 mm, such as between 55-65 mm.

Fig. 5c illustrates the third primary gel pack 140, which, as mentioned previously, has a third primary shape 141. The third primary shape 141 is a substantially rectangular shape. The third primary shape 141 has a first edge side 142 and an opposite second edge side 143. The third primary shape 141 has a third edge side 144 between the first edge side 142 and the second edge side 143. The third primary shape 141 has a fourth edge side 145 between the first edge side 142 and the second edge side 143. The fourth edge side 145 is opposite the third edge side.

The first edge side 142 and the second edge side 143 are parallel. The third edge side 144 and the fourth edge side 145 are parallel. The first edge side 142 and the second edge side 143 are perpendicular to the third edge side 144 and the fourth edge side 145.

The first edge side 142 and the second edge side 143 may have a length between 90-120 mm, such as between 100-110 mm. The third edge side 144 and the fourth edge side 145 may have a length between 70-110 mm, such as between 85-95 mm.

Fig. 5d illustrates the third secondary gel pack 150, which, as mentioned previously, has a third secondary shape 151. The third secondary shape 151 is a substantially rectangular shape. The third secondary shape 151 has a first edge side 152 and an opposite second edge side 153. The third secondary shape 151 has a third edge side 154 between the first edge side 152 and the second edge side 153. The third secondary shape 151 has a fourth edge side 155 between the first edge side 152 and the second edge side 153. The fourth edge side 155 is opposite the third edge side 154.

The first edge side 152 and the second edge side 153 are parallel. The third edge side 154 and the fourth edge side 155 are parallel. The first edge side 152 and the second edge side 153 are perpendicular to the third edge side 154 and the fourth edge side 155.

The first edge side 152 and the second edge side 153 may have a length between 140-170 mm, such as between 150-160 mm. The third edge side 154 and the fourth edge side 155 may have a length between 70-110 mm, such as between 85-95 mm.

Fig. 5e illustrates the third tertiary gel pack 160, which, as mentioned previously, has a third tertiary shape 161. The third tertiary shape 161 is a substantially rectangular shape. The third tertiary shape 161 has a first edge side 162 and an opposite second edge side 163. The third tertiary shape 161 has a third edge side 164 between the first edge side 162 and the second edge side 163. The third tertiary shape 161 has a fourth edge side 165 between the first edge side 162 and the second edge side 163. The fourth edge side 165 is opposite the third edge side 164.

The first edge side 162 and the second edge side 163 are parallel. The third edge side 164 and the fourth edge side 165 are parallel. The first edge side 162 and the second edge side 163 are perpendicular to the third edge side 164 and the fourth edge side 165.

The first edge side 162 and the second edge side 163 may have a length between 190-235 mm, such as between 205-220 mm. The third edge side 164 and the fourth edge side 165 may have a length between 70-110 mm, such as between 85-95 mm.

Fig. 5f illustrates the fourth gel pack 170, which, as mentioned previously, has a fourth shape 171. The fourth shape 171 is a kidney shape or bean shape. The fourth shape 171 has a first edge side 172 and an opposite second edge side 173. The first edge side 172 has a convex curvature. The second edge side 173 has a concave curvature. In some examples, the second edge side 173 may be substantially straight. The convex curvature of the first edge side 172 facilitates the fourth gel pack 170 fitting over the nose of the user. The radius of curvature of the first edge side 172 may be smaller than the radius of curvature of the second edge side 173. The radius of curvature of the first edge side 172 may be between 70-100 mm, such as between 80-90 mm. The radius of curvature of the second edge side 173 may be more than 500 mm, such as more than 800 mm.

A height H1 of the fourth shape 171 may be measured from the second edge side 173 and denoting the maximum distance of the fourth shape 171 perpendicular to the second edge side 173. The height H1 of the fourth shape 171 may be between 50-95 mm, such as between 65-80 mm.

A width W1 of the fourth shape 171 may denote the maximum distance of the fourth shape 171 measured perpendicular to the height H1, e.g. parallel to the second edge side 173. The width W1 may be between 150-210 mm, such as between 170-190 mm.

The disclosure has been described with reference to a preferred embodiment. However, the scope of the invention is not limited to the illustrated embodiment, and alterations and modifications can be carried out without deviating from the scope of the invention.

Throughout the description, the use of the terms "first", "second", "third", "fourth", "primary", "secondary", "tertiary" etc. does not imply any particular order or importance, but are included to identify individual elements. Furthermore, the labelling of a first element does not imply the presence of a second element and vice versa.

### LIST OF REFERENCES

- 4: user
- 6: head

- 10: headwear
- 12: inside fabric layer
- 14: outside fabric layer
- 16: pockets
- 18: pocket opening
- 20: inside overlap
- 22: straps

- 50: gel packs
- 52: gel or liquid
- 54: first side
- 56: second side
- 58: rim
- 60: dividing path
- 62a, 62b: sub-compartment
- 64: label or icon

- 100: first primary gel pack
- 101: first primary shape
- 102: first edge side of first primary shape
- 103: second edge side of first primary shape
- 104: third edge side of first primary shape
- 105: fourth edge side of first primary shape

- 110: first secondary gel pack
- 111: first secondary shape
- 112: first edge side of first secondary shape
- 113: second edge side of first secondary shape
- 114: third edge side of first secondary shape
- 115: fourth edge side of first secondary shape
- 116: fifth edge side of first secondary shape
- 120: second primary gel pack
- 130: second secondary gel pack

- 140: third primary gel pack
- 141: third primary shape
- 142: first edge side of third primary shape
- 143: second edge side of third primary shape
- 144: third edge side of third primary shape
- 145: fourth edge side of third primary shape

- 150: third secondary gel pack
- 151: third secondary shape
- 152: first edge side of third secondary shape
- 153: second edge side of third secondary shape
- 154: third edge side of third secondary shape
- 155: fourth edge side of third secondary shape

- 160: third tertiary gel pack
- 161: third tertiary shape
- 152: first edge side of third tertiary shape
- 153: second edge side of third tertiary shape
- 154: third edge side of third tertiary shape
- 155: fourth edge side of third tertiary shape

- 170: fourth gel pack
- 171: fourth shape
- 172: first edge side of fourth shape
- 173: second edge side of fourth shape

## Claims

1. A headwear comprising a plurality of gel packs for cooling or heating of selective regions of a user's head,
the headwear has an inside fabric layer and an opposite outside fabric layer, wherein the inside fabric layer is adapted to be arranged towards the head of the user when wearing the headwear, the headwear further comprising a plurality of pockets formed between the inside fabric layer and the outside fabric layer, each of the plurality of pockets being adapted to contain a corresponding gel pack of the plurality of gel packs,
each of the plurality of gel packs comprises a liquid or a gel having a specific heat capacity sufficient to provide cooling or heating after being cooled or heated, and wherein each of the plurality of gel packs have a first side and an opposite second side, the first side joining the opposite second side along a rim defining a substantially planar shape of the respective gel pack,
the plurality of gel packs comprising a first primary gel pack and a first secondary gel pack,
wherein the substantially planar shape of the first primary gel pack is a first primary shape and the substantially planar shape of the first secondary gel pack is a first secondary shape different than the first primary shape, and wherein the first primary shape and the first secondary shape are not rectangular.

2. Headwear according to any of the preceding claims, wherein the first primary shape has a first edge side with a convex curvature and an opposite second edge side with a concave curvature.

3. Headwear according to any of the preceding claims, wherein the first secondary shape has a first edge side being substantially straight and an opposite second edge side being non-parallel with the first edge side.

4. Headwear according to claim 3, wherein the first secondary shape has a third edge side between the first edge side and the second edge side of the first secondary shape, the third edge side being substantially straight, the third edge side being substantially perpendicular to the first edge side of the first secondary shape.

5. Headwear according to any of claims 3-4, wherein the first secondary shape has a fourth edge side between the first edge side and the second edge side of the first secondary shape, the fourth edge side being substantially straight, the fourth edge side being non-parallel with the third edge side of the first secondary shape.

6. Headwear according to claim 5, wherein the first secondary shape has a fifth edge side between the first edge side and the fourth edge side of the first secondary shape, the fifth edge side being concave.

7. Headwear according to any of the preceding claims, wherein the plurality of gel packs comprises a second primary gel pack and a second secondary gel pack, and wherein the substantially planar shape of the second primary gel pack is the first primary shape and the substantially planar shape of the second secondary gel pack is the first secondary shape.

8. Headwear according to any of the preceding claims, wherein the plurality of gel packs comprises a third primary gel pack, and wherein the substantially planar shape of the third primary gel pack is a third primary shape being substantially rectangular, and
optionally wherein the plurality of gel packs comprises a third secondary gel pack and wherein the substantially planar shape of the third secondary gel pack is a third secondary shape being substantially rectangular, and/or
optionally wherein the plurality of gel packs comprises a third tertiary gel pack and wherein the substantially planar shape of the third tertiary gel pack is a third tertiary shape being substantially rectangular.

9. Headwear according to any of the preceding claims, wherein the plurality of gel packs comprises a fourth gel pack, and wherein the substantially planar shape of the fourth gel pack is a fourth shape different than the first primary shape and the first secondary shape, optionally the fourth shape has a first edge side with a convex curvature and an opposite second edge side with a concave curvature.

10. Headwear according to any of the preceding claims, wherein one or more of the plurality of gel packs, such as the first primary gel pack and/or the first secondary gel pack, comprise one or more dividing paths subdividing the respective gel pack into two or more sub-compartments, such as two sub-compartments and/or three sub-compartments, wherein the one or more dividing paths restrict flow of the liquid or gel between two adjacent sub-compartments.

11. Headwear according to any of the preceding claims, wherein the liquid or gel of the plurality of gel packs has a specific heat capacity of at least 2500 J/(kg·K).

12. Headwear according to any of the preceding claims, wherein the liquid or gel of the plurality of gel packs is a hydrogel and/or wherein the liquid or gel of the plurality of gel packs comprises more than 70% water and/or more than 15% propylene glycol.

13. Headwear according to any of the preceding claims, wherein each of the plurality of pockets has a pocket opening through the inside fabric layer, allowing insertion and removal of the gel packs from their respective pocket, optionally wherein each of the plurality of pockets has an inside overlap at the pocket opening adapted to restrict removal of the gel pack from the pocket.

14. Headwear according to any of the preceding claims, wherein the outside fabric layer is made of neoprene, and/or wherein the inside fabric layer comprises graphene.

15. An assembly of gel packs for cooling or heating of selective regions of a user's head, the assembly comprising a plurality of gel packs, wherein each of the plurality of gel packs comprises a liquid or a gel having a specific heat capacity sufficient to provide cooling or heating after being cooled or heated, and wherein each of the plurality of gel packs have a first side and an opposite second side, the first side joining the opposite second side along a rim defining a substantially planar shape of the respective gel pack,
the plurality of gel packs comprising a first primary gel pack and a first secondary gel pack,
wherein the substantially planar shape of the first primary gel pack is a first primary shape and the substantially planar shape of the first secondary gel pack is a first secondary shape different than the first primary shape.
